# EUROPEAN PATENT SPECIFICATION

(11) **EP 0 689 444 B1**
(45) Date of publication and mention of the grant of the patent: **07.07.1999**
(21) Application number: 94909996.4
(22) Date of filing: 22.03.1994
(51) Int. Cl.: A61K 31/71, A61K 9/08, A61K 47/12, A61K 47/14

(54) **TYLOSIN CONTAINING SUSTAINED RELEASE VETERINARY COMPOSITIONS**
TYLOSIN ENTHALTENDE TIERAERZTLICHE ZUBEREITUNGEN MIT VERZOEGERTER WIRKSTOFFABGABE
COMPOSITIONS VETERINAIRES A LIBERATION PROLONGEE CONTENANT DE LA TYLOSINE

(30) Priority: 24.03.1993 GB 9306106
(43) Date of publication of application: 03.01.1996
(73) Proprietor: NORBROOK LABORATORIES LIMITED, Newry BT35 6JP (GB)
(72) Inventor: PATTERSON, Alan, Belfast BT4 2NQ (GB); HOLMES, Drew, Newcastle, Co Down BT33 0SL (GB)
(74) Representative: Ede, Eric
(86) International application number: GB9400578
(87) International publication number: WO9421267

(56) References cited:
- EP-A- 0 091 767
- EP-A- 0 236 901
- GB-A- 2 105 589

## Description

This invention relates to a pharmaceutical preparation and in particular to a veterinary preparation for intramuscular and subcutaneous administration of Tylosin.

Tylosin is a member of the macrolide group of antibiotics which is indicated clinically for the treatment of many bacterial infections in beef cattle, non-lactating dairy cattle and swine.

Tylosin is available as a parenteral product containing 50 mg/ml or 200 mg/ml active ingredient typically administered once daily for a three day period. However such preparations provide quick onset and short duration of action and are associated with irritancy at the site of injection. Thus there is a clinical need for a Tylosin preparation which provides a sustained release of Tylosin over a period of days and may be administered with minimal adverse tissue reactions. An object of this invention is to obviate or mitigate the aforesaid disadvantages.

Accordingly this invention aims to provide a veterinary preparation for intramuscular and subcutaneous administration comprising Tylosin in a system which promotes sustained release of the active ingredient.

According to this invention there is provided a sustained release composition, comprising Tylosin and an oily vehicle containing a gelling agent, e.g. an aluminium salt of a fatty acid. Preferably the oily vehicle is a low viscosity polyol diester of a short chain naturally derived fatty acid, typically propylene glycol dicaprylate/dicaprate. Preferably this vehicle is that which is obtainable under the trade name Neobee Oil. The oily vehicle is preferably present in an amount of from 70-90%, most preferably 84% by weight of the composition.

The gelling agent present is typically aluminium distearate. The aluminium distearate may be present in an amount of from 0.75-1.25%, but most preferably 0.94%, by weight. Other stearates may also be used to replace aluminium distearate, namely aluminium monostearate or aluminium tristearate. Preferably the aluminium monostearate is present in an amount of from 1-3% but most preferably about 1.8% by weight. Alternatively, an equivalent amount of aluminium tristearate should be present.

This invention further provides a method for incorporation of an active ingredient such as Tylosin into the oily vehicle.

Preferably, Tylosin is present in an amount from 3-30% but most preferably approximately 15% by weight per unit volume. The particle range of the active ingredient is preferably controlled so that 90% of particles have a major dimension of less than 25 µm as measured using the Coulter Counter technique or an equivalent dimension when measured using alternative methods. More preferably the particle size should be less than 10 µm.

The invention also provides a process for preparing a veterinary preparation for intramuscular and subcutaneous administration as hereinbefore defined comprising the steps of:-

Mixing a pharmaceutically acceptable (sterile) vehicle and an agent for sustained release and applying sufficient heat to permit adequate mixing, allowing the mixture to cool to form a gel base, and adding Tylosin to form a suspension thereof in the gel base.

The invention may be more clearly understood from the following description of a formulation thereof given by way of example only:

| **FORMULATION** | **QUANTITY (g/l)** |
|---|---|
| Tylosin | 150.00 |
| Aluminium distearate | 9.35 |
| Neobee Oil M20 | 840.65 |

The Neobee Oil is placed in a stainless steel pressure vessel in an aseptic area. The contents of the vessel are then heated to 160°C and the temperature is maintained for one hour to sterilise the contents of the vessel. This is allowed to cool and, when the temperature reaches 120°C, the aluminium distearate is added with gentle stirring until dissolution has occurred. The solution is then allowed to cool without further stirring until ambient temperature is reached. At this stage Tylosin is added and suspended aseptically. To achieve the required particle size of Tylosin, the product may be passed through a conventional milling device. Preferably this milling device is a bead mill e.g. that sold under the name Dyno Mill. The final product is a stable off-white/cream suspension which can be used for the prophylaxis and treatment of cattle and pigs infected by micro-organisms sensitive to Tylosin. In general, the product is best administered by intramuscular injection.

The optimal concentration of Tylosin is 150 mg/ml. This has been chosen after consideration of the dose required to produce the required therapeutic effect after a single administration.

In the selection of appropriate oily vehicles, several candidates were initially examined for their suitability in this preparation. Resultant from this a polyol diester of short chain naturally derived fatty acids, propylene glycol dicaprylate/dicaprate was chosen. This oil has low viscosity, low setting point, and is therefore a useful vehicle for parenteral products.

Aluminium distearate is used to gel oily vehicles and, in so doing, to promote sustained release of Tylosin at the site of injection. The process of heating the vehicle, addition of aluminium stearate and allowing the product to cool results in a stable thixotropic gel which both ensures the retention of Tylosin in suspension and accuracy of dosage. The amount of aluminium distearate used is chosen such that there is facilitation of formation of suspension and syringeability.

The particle size range of Tylosin used is of vital importance as it ensures good resuspension of product and contributes to the sustained activity of the product. For these reasons it is preferable that 90% of particles have a major dimension of less than 25 µm as measured using the Coulter Counter technique or an equivalent dimension when measured using alternative techniques. Indeed it is more preferable that the particle size is less than 10 µm.

The efficacy of invention will now be further described by way of the following test examples:

### Example 1

A 15% (w/v) Tylosin in Neobee oil (identified hereinafter as "Tylosin LA") was prepared by procedures following usual industry practice. Administration thereof by intramuscular injection to cattle at a dose rate of 20 mg/kg bodyweight was carried out to enable a study of the plasma levels and hence the efficacy of the product to be made.

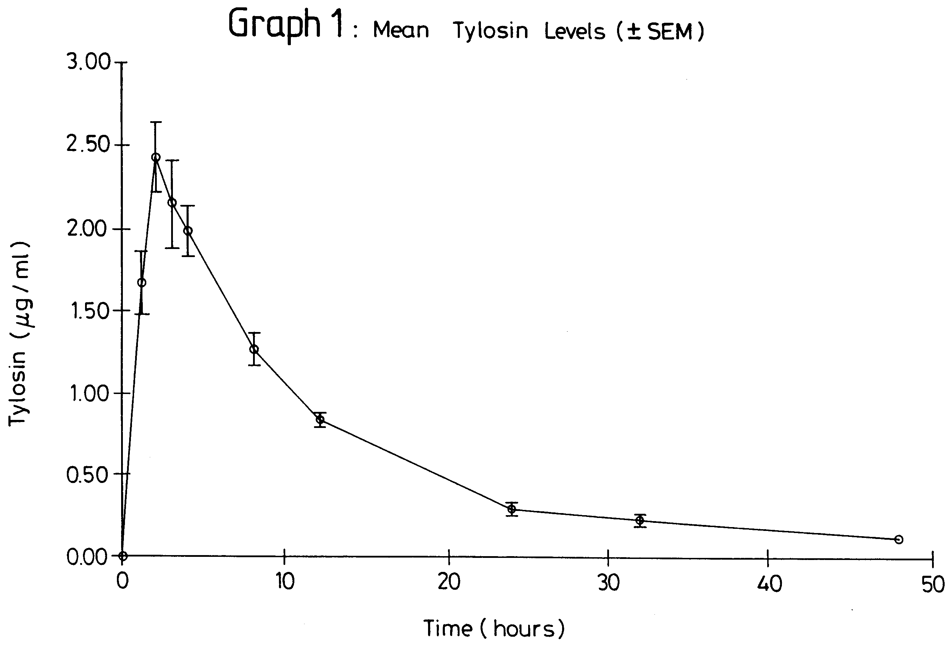

The tylosin values at each timepoint were meaned, the standard errors determined and the results presented in Table 1 and also graphically (Graph 1). The mean Cₘₐₓ was 2.612 ± 0.249 µg/ml, achieved after 3 ± 0.5 hours. The mean area under the curve (AUC) was 31.32 ± 1.84 µg/ml.hr. The half-life of elimination was found to be 10.01 ± 1.7 hours.

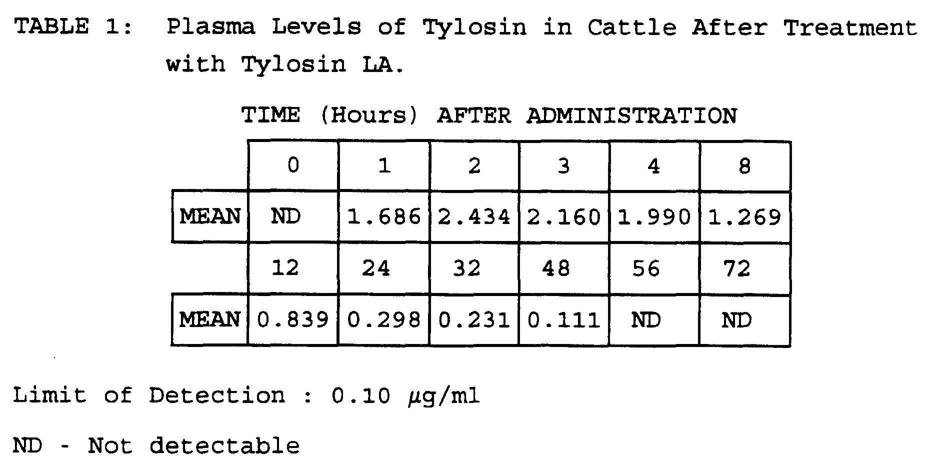

### Example 2

An efficacy study in pigs following the intramuscular administration of Tylosin LA and a proprietary Tylosin product in the treatment of induced respiratory tract infections was carried out.
This study demonstrated the efficacy of parenterally administered Tylosin in the treatment of an induced, acute, lower respiratory tract infection of Pasteurella multocida and Mycoplasma hypopneumonia in pigs. The study involved a total of 40 pigs.

Three treatment groups were involved in this study:
1. Tylosin LA at 20 mg/kg bodyweight on a single occasion.
2. Tylosin LA at 30 mg/kg bodyweight on a single occasion.
3. Proprietary Tylosin product (50 mg/ml) at 3 daily 10 mg/kg bodyweight doses.

A summary of the results for the mean body temperature and the mean clinical scores are presented in Tables 2 and 3 respectively.

A repeatable artificial infection model of severe clinical pneumonia was induced in the pigs. Treatment with one of 3 dose regimes was utilised to compare the efficacy of Tylosin LA and the proprietary Tylosin product. The clinical response to treatment was excellent in that all animals which survived the first 24 hours after inoculation showed significant clinical improvements similar to the field treatment of natural clinical cases with antibiotic therapy.

The use of weighted scoring system has shown that a single administration of Tylosin LA at either of the dose rates used (20 mg/kg or 30 mg/kg administered on a single occasion) demonstrated statistically significant improvements in recovery rates when compared to a 3 day course of treatment with the proprietary Tylosin product. It can therefore be concluded that Tylosin LA in a single administration of supply will be at least as effective as the course of treatment using proprietary Tylosin product when used in the treatment of pneumonia.

**TABLE 2**

| Mean Body Temperature | | | |
|---|---|---|---|
| TIME (Hours From First Administration) | TYLOSIN LA 20 mg/kg (n = 11) | TYLOSIN LA 30 mg/kg (n = 12) | PROPRIETARY PRODUCT (50 mg/ml) 3 x 10 mg/kg (n = 12) |
| | MEAN | MEAN | MEAN |
| -96 | 102.32 | 102.08 | 102.09 |
| -88 | 102.06 | 102.12 | 102.09 |
| -72 | 102.18 | 102.09 | 102.22 |
| -64 | 102.11 | 102.08 | 102.22 |
| -48 | 102.17 | 102.18 | 102.09 |
| -24 | 102.20 | 102.09 | 102.18 |
| -16 | 102.14 | 102.25 | 102.18 |
| 0+ | 104.63 | 104.68 | 104.38 |
| 4 | 104.33 | 104.65 | 104.47 |
| 8 | 103.75 | 103.97 | 103.90 |
| 12 | 103.38 | 103.62 | 103.62 |
| 24++ | 103.20 | 103.20 | 103.53 |
| 32 | 103.07 | 103.07 | 103.60 |
| 48+++ | 102.69 | 102.82 | 103.40 |
| 56 | 102.96 | 102.90 | 103.48 |
| 72 | 102.78 | 102.33 | 102.97 |
| 96 | 102.56 | 102.37 | 102.87 |
| 120 | 102.51 | 102.30 | 102.72 |
| 192 | 102.38 | 102.42 | 102.83 |
| 240 | 102.40 | 102.30 | 102.78 |
| 288 | 102.24* | 102.29 | 102.93 |
| 360 | 102.49* | 102.25 | 102.43 |

| | | | |
|---|---|---|---|
| * n = 10 for these timepoints. | | | |
| + Administration of Tylosin LA and first administration of Proprietary Product. | | | |
| ++ Second administration of Proprietary Product. | | | |
| +++ Third administration of Proprietary Product. | | | |

**TABLE 3**

| Mean Clinical Scores | | | |
|---|---|---|---|
| TIME (Hours From First Administration) | TYLOSIN LA 20 mg/kg (n = 11) | TYLOSIN LA 30 mg/kg (n = 12) | PROPRIETARY PRODUCT (50 mg/ml) 3 x 10 mg/kg (n = 12) |
| | MEAN | MEAN | MEAN |
| -16 | 0 | 0 | 0 |
| 0+ | 36.2 | 35.9 | 35.0 |
| 4 | 34.3 | 35.7 | 34.8 |
| 8 | 29.9 | 31.4 | 30.0 |
| 12 | 26.4 | 29.8 | 30.4 |
| 24++ | 22.1 | 24.3 | 27.1 |
| 32 | 21.8 | 22.3 | 28.9 |
| 48+++ | 17.3 | 18.8 | 24.6 |
| 56 | 18.2 | 19.3 | 24.9 |
| 72 | 15.0 | 16.8 | 20.1 |
| 96 | 12.5 | 17.3 | 21.0 |
| 120 | 11.2 | 13.8 | 17.5 |
| 192 | 9.3 | 10.5 | 14.3 |
| 240 | 8.8 | 9.2 | 11.9 |
| 288 | 8* | 8.9 | 11.3 |
| 360 | 5.7* | 7.7 | 8.4 |

| | | | |
|---|---|---|---|
| * n = 10 for these timepoints. | | | |
| + Administration of Tylosin LA and first administration of Proprietary Product. | | | |
| ++ Second administration of Proprietary Product. | | | |
| +++ Third administration of Proprietary Product. | | | |

## Claims

1. A sustained release composition, comprising Tylosin and an oily vehicle containing a gelling agent.

2. A sustained release composition according to claim 1 wherein the gelling agent is an aluminium salt of a fatty acid.

3. A sustained release composition according to claim 2 wherein the fatty acid is stearic acid.

4. A sustained release composition according to claim 1 or claim 2 wherein the oily vehicle is a low viscosity polyol diester of a short chain naturally derived fatty acid.

5. A sustained release composition according to claim 4 wherein the oily vehicle is propylene glycol dicaprylate/dicaprate.

6. A sustained release composition according to claim 1 or claim 2 wherein the oily vehicle is present in an amount of from 70-90% by weight of the composition.

7. A sustained release composition according to claim 1 or claim 2 wherein the oily vehicle is present in an amount of 84% by weight of the composition.

8. A sustained release composition according to claim 3 wherein the gelling agent is aluminium distearate.

9. A sustained release composition according to claim 8 wherein the aluminium distearate is present in an amount of from 0.75-1.25% by weight of the composition.

10. A sustained release composition according to claim 9 wherein the aluminium distearate is present in an amount of from about 0.94% by weight of the composition.

11. A sustained release composition according to claim 3 wherein the gelling agent is selected from the group consisting of aluminium monostearate, aluminium distearate and aluminium tristearate.

12. A sustained release composition according to claim 11 wherein aluminium monostearate is present in an amount of from 1-3% by weight.

13. A sustained release composition according to claim 11 wherein aluminium monostearate is present in an amount of about 1.8% by weight.

14. A sustained release composition according to claim 1 wherein the composition substantially consists of 840.65 mg/ml propylene glycol dicaprylate/dicaprate, 9.35 mg/ml aluminium distearate, and 150 mg/ml Tylosin.

15. A method for incorporation of the active ingredient Tylosin into an oily vehicle comprising the addition of a gelling agent to said oily vehicle, controlling the particle size of said active ingredient so that 90% of particles have a major dimension of less than 25 µm as measured using the Coulter Counter technique and mixing said active ingredient into said oily vehicle.

16. A method according to claim 15 wherein the active ingredient Tylosin is present in an amount from 3-30% by weight per unit volume.

17. A method according to claim 15 wherein the particle range of the active ingredient Tylosin is controlled so that 90% of particles have a major dimension less than 10 µm.

18. A process for preparing a veterinary preparation for intramuscular and subcutaneous administration comprising the steps of:-
(i) mixing a pharmaceutically acceptable oily vehicle and a gelling agent for conferring sustained release properties and applying sufficient heat to permit adequate mixing,
(ii) allowing the mixture to cool to form a gel base, and
(iii) adding Tylosin to form a suspension thereof in the gel base.

19. A process according to claim 18 wherein the concentration of Tylosin is 150 mg/ml.

20. Use of propylene glycol dicaprylate/dicaprate with a gelling agent in the manufacture of a veterinary composition containing Tylosin as active principle to obtain a parenteral composition exhibiting sustained release of Tylosin for the purposes of countering infection by microorganisms sensitive to Tylosin.

21. Use as claimed in claim 20 wherein the composition contains Tylosin in an amount of from 3 - 30% (by weight per unit volume), and a stearate gelling agent in an amount of from 1 - 3% by weight in the case of aluminium monostearate, or an equivalent amount of aluminium tristearate, in propylene glycol dicaprylate/dicaprate.

22. Use claimed in claim 20 wherein the composition contains Tylosin in an amount of about 150 mg/ml.

23. Use claimed in claim 20 wherein the composition contains 15% (w/v) Tylosin, and the gelling agent is aluminium distearate.

24. A sustained release composition comprising Tylosin and an oily vehicle containing a gelling agent, the Tylosin being present in an amount from 3 to 30% by weight per unit volume and having a particle size range such that 90% of particles have a major dimension of less than 25µm as measured using the Coulter Counter technique, the oily vehicle being present in an amount from 70 to 90% by weight of the composition and being a low viscosity polyol diester of a short chain naturally derived fatty acid, the gelling agent being an aluminium stearate.

25. A sustained release composition according to claim 24, wherein the Tylosin is present in an amount of approximately 15% by weight per unit volume.

26. A sustained release composition according to claim 24 or 25, wherein the particle size of the Tylosin is such that 90% of particles have a major dimension of less than 10µm.

27. A sustained release composition according to claim 24, 25, or 26, wherein the oily vehicle is propylene glycol dicaprylate/dicaprate.

28. A sustained release composition according to claim 24, 25, 26 or 27, wherein the aluminium stearate is aluminium distearate and is present in an amount of from 0.75 to 1.25% by weight.

29. A sustained release composition according to claim 24, 25, 26 or 27, wherein the aluminium stearate is aluminium monostearate present in an amount of from 1 to 3% by weight, or is aluminium tristearate present in an equivalent amount.

## Patentansprüche

1. Zubereitung mit verzögerter Wirkstoffabgabe bestehend aus Tylosin in einem öligen Träger, der ein Geliermittel enthält.

2. Zubereitung mit verzögerter Wirkstoffabgabe gemäss Anspruch 1, bei der das Geliermittel ein Aluminiumsalz einer Fettsäure ist.

3. Zubereitung mit verzögerter Wirkstoffabgabe gemäss Anspruch 2, bei der die Fettsäure Stearinsäure ist.

4. Zubereitung mit verzögerter Wirkstoffabgabe gemäss Anspruch 1 oder 2, bei der der ölige Träger ein niedrig-viskoser Polyoldiester, abgeleitet von einer natürlichen, kurzkettigen Fettsäure ist.

5. Zubereitung mit verzögerter Wirkstoffabgabe gemäss Anspruch 4, bei der der ölige Träger Propylenglykol-Dicaprylat/Dicaprat ist.

6. Zubereitung mit verzögerter Wirkstoffabgabe gemäss Anspruch 1 oder 2, bei der die Zubereitung den öligen Träger mit einem Gehalt von 70 bis 90 Gewichts% enthält.

7. Zubereitung mit verzögerter Wirkstoffabgabe gemäss Anspruch 1 oder 2, bei die Zubereitung den öligen Träger mit einem Gehalt von 84 Gewichts% enthält.

8. Zubereitung mit verzögerter Wirkstoffabgabe gemäss Anspruch 3, bei der das Geliermittel ein Aluminiumdistearat ist.

9. Zubereitung mit verzögerter Wirkstoffabgabe gemäss Anspruch 8, bei der die Zubereitung das Aluminiumdistearat mit einem Gehalt von 0.75 bis 1.25 Gewichts% enthält.

10. Zubereitung mit versögerter Wirkstoffabgabe gemäss Anspruch 9, bei der die Zubereitung das Aluminiumdistearat mit einem Gehalt von etwa 0.94 Gewichts% enthält.

11. Zubereitung mit verzögerter Wirkstoffabgabe gemäss Anspruch 3, bei der das Geliermittel aus einer Gruppe enthaltend Aluminiummonostearate, Aluminiumdistearate und Aluminiumtristearate ausgewählt wird.

12. Zubereitung mit verzögerter Wirkstoffabgabe gemäss Anspruch 11, bei der die Zubereitung das Aluminiummonostearat mit einem Gehalt 1 bis 3 Gewichts% enthält.

13. Zubereitung mit verzögerter Wirkstoffabgabe gemäss Anspruch 11, bei der die Zubereitung das Aluminiummonostearat mit einem Gehalt von etwa 1.8 Gewichts% enthält.

14. Zubereitung mit verzögerter Wirkstoffabgabe gemäss Anspruch 1, bei der die Zubereitung im wesentlichen aus 840.65 mg/ml Propylenglykol-Dicaprylat/Dicaprat, 9.35 mg/ml Aluminiumdistearat und 150 mg/ml Tylosin besteht.

15. Methode zur Einbindung des aktiven Bestandteils Tylosin in einen öligen Träger, die die Zugabe eines Geliermittels zu dem öligen Träger umfasst, die die Teilchengrösse besagter aktiven Zugabe so überwacht, dass 90% der Teilchen eine grössere Abmessung von unter 25 µm aufweisen, gemessen nach Coulter-Counter-Technik, und die die besagte aktive Zugabe in besagten öligen Träger mischt.

16. Methode nach Anspruch 15, bei der die aktive Zugabe Tylosin mit einem Gehalt von 3 bis 30 Gewichts% pro Volumeneinheit vorliegt.

17. Methode nach Anspruch 15, bei dem der Teilchenbereich der aktiven Zugabe Tylosin so überwacht wird, dass 90% der Teilchen eine grössere Abmessung von unter 10 µm aufweisen.

18. Verfahren zur Herstellung einer tierärztlichen Zubereitung für die intramuskuläre und subkutane Verabreichung, das die folgenden Schritte umfasst:
i) Mischen eines pharmazeutisch akzeptablen öligen Trägers und einem Geliermittel zum Erhalten der Eigenschaften verzögerter Wirkstoffabgabe und Anwendung von ausreichender, ein adequates Mischen erlaubender Wärme,
ii) Kühlen der Mischung zur Bildung einer Gel-Basis, und
iii) Hinzufügen von Tylosin, zur Bildung einer Suspension in der Gel-Basis.

19. Verfahren nach Anspruch 18, bei dem die Konzentration von Tylosin 150 mg/ml ist.

20. Verwendung von Propylenglykol-Dicaprylat/Dicaprat mit einem Geliermittel zur Herstellung einer tierärztlichen Zubereitung mit Tylosin als aktiven Grundstoff für eine parenteral anzuwendende Zubereitung, die die verzögerte Freigabe von Tylosin aufweist, um Infektionen entgegenzuwirken, die durch auf Tylosin empfindliche Mikroorganismen hervorgerufen werden.

21. Verwendung wie in Anspruch 20 beansprucht, bei der die Zubereitung Tylosin mit einem Gehalt von 3 bis 30 Gewichts% je Volumeneinheit enthält, und ein Stearat-Geliermittel mit einem Gehalt von 1 bis 3 Gewichts% im Falle von Aluminiummonostearat, oder einer äquivalenten Menge von Aluminiumtristearat bei Propylenglykol-Dicaprylat/Dicaprat.

22. Verwendung wie in Anspruch 20 beansprucht, bei der die Zubereitung Tylosin mit einem Gehalt von etwa 150 mg/ml enthält.

23. Verwendung wie in Anspruch 20 beansprucht, bei der die Zubereitung 15% (Gew/Vol) Tylosin enthält und das Geliermittel Aluminiumdistearat ist.

24. Zubereitung mit verzögerter Wirkstoffabgabe, die Tylosin und einen Geliermittel enthaltenden öligen Träger enthält, wobei das Tylosin mit einem Gehalt von 3 bis 30 Gewichts% je Volumeneinheit vorliegt, wobei der Bereich der Teilchengrösse so liegt, dass 90% der Teilchen eine grössere Abmessung von unter 25 m aufweisen, gemessen mit Coulter-Counter-Technik, wobei der in der Zubereitung mit einem Gehalt von 70 bis 90 Gewichts% vorliegende ölige Träger ein niedrig-viskoser Polyoldiester, abgeleitet von einer natürlichen, kurz-kettigen Fettsäure ist, und wobei das Geliermittel ein Aluminiumstearat ist.

25. Zubereitung mit verzögerter Wirkstoffabgabe gemäss Anspruch 24, bei der das Tylosin mit einem Gehalt von annähernd 15 Gewichts% je Volumeneinheit vorliegt.

26. Zubereitung mit verzögerter Wirkstoffabgabe gemäss Anspruch 24 oder 25, bei der die Teilchengrösse des Tylosin so ist, dass 90% der Teilchen eine grössere Abmessung von unter 10 µm aufweisen.

27. Zubereitung mit verzögerter Wirkstoffabgabe gemäss Anspruch 24, 25 oder 26, bei der der ölige Träger Propylenglykol-Dicaprylat/Dicaprat ist.

28. Zubereitung mit verzögerter Wirkstoffabgabe gemäss Anspruch 24, 25, 26 oder 27, bei der das Aluminiumstearat Aluminiumdistearat ist und mit einem Gehalt von 0.75 bis 1.25 an Gewichts% vorliegt.

29. Zubereitung mit verzögerter Wirkstoffabgabe gemäss Anspruch 24, 25, 26 oder 27, bei der das Aluminiumstearat Aluminiummonostearat ist und mit einem Gehalt von 1 bis 3 Gewichts% vorliegt oder Aluminiumtristearat und in einer äquivalenten Menge vorhanden ist.

## Revendications

1. Composition retard, comprenant de la tylosine et un véhicule huileux contenant un agent de gélification.

2. Composition retard selon la revendication 1, dans laquelle l'agent de gélification est un sel d'aluminium d'un acide gras.

3. Composition retard selon la revendication 2, dans laquelle l'acide gras est l'acide stéarique.

4. Composition retard selon la revendication 1 ou 2, dans laquelle le véhicule huileux est un diester de polyol à faible viscosité d'un acide gras naturel à chaîne courte.

5. Composition retard selon la revendication 4, dans laquelle le véhicule huileux est le dicaprylate/dicaprate de propylèneglycol.

6. Composition retard selon la revendication 1 ou 2, dans laquelle le véhicule huileux est présent en une quantité de 70 à 90 % en poids par rapport à la composition.

7. Composition retard selon la revendication 1 ou 2, dans laquelle le véhicule huileux est présent en une quantité de 84 % en poids par rapport à la composition.

8. Composition retard selon la revendication 3, dans laquelle l'agent de gélification est le distéarate d'aluminium.

9. Composition retard selon la revendication 8, dans laquelle le distéarate d'aluminium est présent en une quantité de 0,75 à 1,25 % en poids par rapport à la composition.

10. Composition retard selon la revendication 9, dans laquelle le distéarate d'aluminium est présent en une quantité d'environ 0,94 % en poids par rapport à la composition.

11. Composition retard selon la revendication 3, dans laquelle l'agent de gélification est choisi dans le groupe constitué du monostéarate d'aluminium, du distéarate d'aluminium et du tristéarate d'aluminium.

12. Composition retard selon la revendication 11, dans laquelle le monostéarate d'aluminium est présent en une quantité de 1 à 3 % en poids.

13. Composition retard selon la revendication 11, dans laquelle le monostéarate d'aluminium est présent en une quantité d'environ 1,8 % en poids.

14. Composition retard selon la revendication 1, dans laquelle la composition est pour l'essentiel constituée de 840,65 mg/l de dicaprylate/dicaprate de propylèneglycol, de 9,35 mg/ml de distéarate d'aluminium et de 150 mg/ml de tylosine.

15. Procédé pour incorporer le principe actif tylosine dans un véhicule huileux, qui comprend l'addition d'un agent de gélification audit véhicule huileux, la régulation de la granulométrie dudit principe actif de façon que 90 % des particules aient une dimension principale inférieure à 25 µm, telle que mesurée par la technique Coulter Counter, ledit principe actif étant ensuite mélangé dans ledit véhicule huileux.

16. Procédé selon la revendication 15, dans lequel le principe actif tylosine est présent en une quantité de 3 à 30 % en poids par unité de volume.

17. Procédé selon la revendication 15, dans lequel l'intervalle granulométrique du principe actif tylosine est régulé de façon que 90 % des particules aient une dimension principale inférieure à 10 µm.

18. Procédé pour préparer une préparation vétérinaire pour administration intramusculaire et sous-cutanée, qui comprend les étapes consistant :
(i) à mélanger un véhicule huileux acceptable d'un point de vue pharmaceutique et un agent de gélification pour conférer des propriétés de libération retardée, et à appliquer une chaleur suffisante pour permettre une opération appropriée de mélange,
(ii) à permettre au mélange de se refroidir pour former une base de gel, et
(iii) à ajouter de la tylosine pour en former une suspension dans la base de gel.

19. Procédé selon la revendication 18, dans lequel la concentration de tylosine est de 150 mg/ml.

20. Utilisation de dicaprylate/dicaprate de propylèneglycol avec un agent de gélification lors de la fabrication d'une composition vétérinaire contenant de la tylosine en tant que principe actif, pour obtenir une composition parentérale présentant une libération retardée de tylosine, dans le but de contrecarrer une infection par des microorganismes sensibles à la tylosine.

21. Utilisation selon la revendication 20, dans laquelle la composition contient de la tylosine en une quantité de 3 à 30 % (en poids par unité de volume) et un agent de gélification de type stéarate en une quantité de 1 à 3 % en poids dans le cas du monostéarate d'aluminium, ou une quantité équivalente de tristéarate d'aluminium dans du dicaprylate/dicaprate de propylèneglycol.

22. Utilisation selon la revendication 20, dans laquelle la composition contient de la tylosine en une quantité d'environ 150 mg/ml.

23. Utilisation selon la revendication 20, dans laquelle la composition contient 15 % (m/v) de tylosine, et l'agent de gélification est le distéarate d'aluminium.

24. Composition retard comprenant de la tylosine et un véhicule huileux contenant un agent de gélification, la tylosine étant présente en une quantité de 3 à 30 % en poids par unité de volume, et ayant un intervalle granulométrique tel que 90 % des particules aient une dimension principale inférieure à 25 µm, telle que mesurée par la technique Coulter Counter, le véhicule huileux étant présent en une quantité de 70 à 90 % en poids par rapport à la composition et étant un diester de polyol à faible viscosité d'un acide gras naturel à courte chaîne, l'agent de gélification étant un stéarate d'aluminium.

25. Composition retard selon la revendication 24, dans laquelle la tylosine est présente en une quantité d'environ 15 % en poids par unité de volume.

26. Composition retard selon la revendication 24 ou 25, dans laquelle la granulométrie de la tylosine est telle que 90 % des particules ont une dimension principale inférieure à 10 µm.

27. Composition retard selon la revendication 24, 25 ou 26, dans laquelle le véhicule huileux est le dicaprylate/dicaprate de propylèneglycol.

28. Composition retard selon la revendication 24, 25, 26 ou 27, dans laquelle le stéarate d'aluminium est le distéarate d'aluminium et est présent en une quantité de 0,75 à 1,25 % en poids.

29. Composition retard selon la revendication 24, 25, 26 ou 27, dans laquelle le stéarate d'aluminium est le monostéarate d'aluminium, présent en une quantité de 1 à 3 % en poids, ou encore est le tristéarate d'aluminium, présent en une quantité équivalente.
